(19) Europäisches Patentamt
European Patent Office
Office européen des brevets

(11) **EP 1 335 892 B1**

(12) **EUROPÄISCHE PATENTSCHRIFT**

(45) Veröffentlichungstag und Bekanntmachung des Hinweises auf die Patenterteilung:
**17.05.2006  Patentblatt 2006/20**

(21) Anmeldenummer: **01978426.3**

(22) Anmeldetag: **17.10.2001**

(51) Int Cl.:
**C07C 51/215** (2006.01)

(86) Internationale Anmeldenummer:
**PCT/EP2001/012001**

(87) Internationale Veröffentlichungsnummer:
**WO 2002/038526 (16.05.2002 Gazette 2002/20)**

(54) **VERFAHREN ZUR HERSTELLUNG VON ESSIGSÄURE**

METHOD FOR PRODUCING ACETIC ACID

PROCEDE DE PRODUCTION D'ACIDE ACETIQUE

(84) Benannte Vertragsstaaten:
**AT BE CH CY DE DK ES FI FR GB GR IE IT LI LU MC NL PT SE TR**

(30) Priorität: **10.11.2000  DE 10055810**

(43) Veröffentlichungstag der Anmeldung:
**20.08.2003  Patentblatt 2003/34**

(73) Patentinhaber: **Celanese International Corporation**
**Dallas,**
**Texas 75381 (US)**

(72) Erfinder:
• **ZEYSS, Sabine**
  **61462 Königstein (DE)**
• **DINGERDISSEN, Uwe**
  **64342 Seeheim-Jugenheim (DE)**
• **BAERNS, Manfred**
  **14195 Berlin (DE)**
• **WOLF, Dorit**
  **60439 Frankfurt (DE)**
• **LINKE, David**
  **12437 Berlin (DE)**

(74) Vertreter: **Ackermann, Joachim et al**
**Postfach 11 13 26**
**60048 Frankfurt am Main (DE)**

(56) Entgegenhaltungen:
**WO-A-00/14047       WO-A-88/04199**
**DE-A- 19 620 542     DE-A- 19 630 832**

**Beschreibung**

Die Erfindung betrifft ein Verfahren zur Herstellung von Essigsäure durch Oxidation von Ethan mit hoher Selektivität und Ausbeute in Wirbelschichtreaktoren.

Die katalytische Gasphasenoxidation von Ethan zu Ethylen und Essigsäure ist seit langem bekannt. Die Umsetzung wird im wesentlichen durch die Wahl der Reaktionsbedingungen beeinflußt, dies gilt insbesondere für die Selektivität der Reaktion. In der Regel entsteht Essigsäure nur als Nebenprodukt, Hauptprodukt ist Ethylen, das unter Umständen bis zum Kohlendioxid weiter oxidiert wird.

In DE 196 30 832 A1, DE 196 20 542 A1, DE 197 45 902 A1, WO 98/47850 und WO 98/47851 wird ein Verfahren zur Herstellung von Essigsäure durch partielle Oxidation von Ethan unter Verwendung von $Mo_aPd_bX_cY_d$ als Katalysatoren im Festbett beschrieben. Der Katalysator zeichnet sich durch seine Stabilität und hohe Selektivität bei der Oxidation von Ethan zur Essigsäure aus. Schwierigkeiten bei der Prozeßführung bereitet allerdings die Tatsache, daß die Oxidation von Ethan zu Essigsäure stark exotherm ist. Insbesondere in größeren Festbettreaktoren, kann die entstehende Reaktionswärme nur unzureichend abgeführt werden. Ein Anstieg der Temperatur im Reaktor und damit ein Absinken der Selektivität der Reaktion ist die Folge.

Um dieses Problem zu umgehen wurde versucht, die Reaktion in einer fluiden Katalysator-Wirbelschicht durchzuführen, wie z. B. in US 5,300,684 beschrieben, wobei zur Wärmeabfuhr direkt Kühlrohre in den Wirbelschichtreaktor eingebracht sind. Eine weiterentwickelte Ausführungsform des Verfahrens wird in WO 00/14047 beschrieben. Nachteilig an den dort beschriebenen Wirbelschichtverfahren ist bislang, daß die Ethanoxidation zu Essigsäure trotzallem mit geringerer Selektivität und geringerer Raumzeitausbeute verläuft als im Festbettreaktor.

Aufgabe der vorliegenden Erfindung war es daher ein Verfahren zur Oxidation von Ethan zu Essigsäure im Wirbelschichtreaktor zur Verfügung zu stellen, das mit höherer Selektivität und damit größerer Ausbeute betrieben werden kann als die bislang beschriebenen Verfahren.

In der vorliegenden Erfindung konnte überraschend gezeigt werden, daß die Selektivität der Ethanoxidation zu Essigsäure von der Größe der Gasblasen im fluiden Katalysatorwirbelschichtbett abhängt. Insbesondere Blasengrößen von <12cm sind vorteilhaft. Besonders bevorzugt sind Blasen mit einem Durchmesser von <5cm.

Über die Steuerung des Gasvolumenstromes bei gegebener durchschnittlicher Partikelgröße des eingesetzten Katalysators kann die Größe der Blasen eingestellt werden. Dabei sind Blasengrößen von <12cm nur durch Verwendung von Katalysatorpartikel mit einem mittleren Durchmesser von kleiner oder gleich $80\mu m$ zu erreichen.

Die vorliegende Erfindung betrifft folglich ein Verfahren zur direkten katalytischen Oxidation von Ethan zu Essigsäure in einer Wirbelschicht enthaltend zu mindestens 70% Katalysatorpartikel mit einem Durchmesser von kleiner oder gleich $80\mu m$. Bevorzugt soll die Wirbelschicht auch kleinere Partikel mit einer Partikelgröße von 10 bis 60 $\mu m$ enthalten, wobei die Verteilung der Partikelgröße derartig sein sollte, daß 10-60% der Partikel einen Durchmesser unter $60\mu m$ haben sollten. Besonders bevorzugt ist eine Verteilung der Partikelgröße derart, daß Katalysatorpartikel in einem Durchmesserbereich von $10-40\mu m$ Breite liegen. In einer besonders bevorzugten Ausführungsform liegt der Durchmesser von 20-50% der Katalysatorpartikel unter 40 $\mu m$.

Die einzustellende Wirbelschichtzahl (Die Wirbelschichtzahl gibt das Verhältnis zwischen eingestellter Gasgeschwindigkeit zu minimaler Fluidisierungsgeschwindigkeit an) muß über 1 liegen. Das heißt die Wirbelschicht muß im fluiden Zustand vorliegen. Bei einem Druck von 16 bar und einer Temperatur von 280 °C ergibt sich eine minimale Fluidisierungsgeschwindigkeit bei einem Partikeldurchmesser von $80\mu m$ von 5,85mm/s. Bevorzugt sollte die Wirbelschichtzahl zwischen 1 und 12 liegen.

Als Katalysator können alle dem Fachmann bekannten Katalysator eingesetzt werden. Als vorteilhaft haben sich Katalysatoren gemäß der allgemeinen Formel (I) erwiesen:

$$M_{0a}Pd_bX_cY_d \qquad (I)$$

wobei X für eines oder mehrere der Elemente ausgewählt aus der Gruppe Cr, Mn, Nb, B, Ta, Ti, V, Te, W und Re steht und Y für eines oder mehrere der Elemente ausgewählt aus der Gruppe B, Al, Ga, In, Pt, Zn, Cd, Bi, Ce, Co, Rh, Ir, Cu, Ag, Au, Fe, Ru, Cs, Mg, Ca, Sr, Ba, Li, K, Na, Rb, Be, Nb, Zr, Hf, Ni, P, Pb, Sb, Si, Sn , Tl, und U steht.

Die Indices a, b, c und d stehen für die Grammatomverhältnisse der entsprechenden Elemente, wobei bezogen auf a=1

b zwischen 0,0001 und 0,01, bevorzugt zwischen 0,0001 bis 0,001

c zwischen 0,1 und 1 und

d zwischen 0 und 1 liegt.

X und/oder Y können auch für mehrere Elemente stehen, wobei gegebenenfalls die Indizes c und d für verschiedene Elemente unterschiedliche Werte annehmen können.

Besonders bevorzugt enthält der Katalysator mindestens eine der folgenden Zusammensetzungen in Kombination mit Sauerstoff:

$$Mo_{1,0}Pd_{0,0005}V_{0,25}Nb_{0,12}$$

$$Mo_{1,00}Pd_{0,0005}V_{0,45}Nb_{0,03}Sb_{0,01}Ca_{0,01}$$

$$Mo_{1,00}Pd_{0,0005}V_{0,45}Nb_{0,03}Sb_{0,01}Ca_{0,01}K_{0,05}$$

$$Mo_{1,00}Pd_{0,00075}V_{0,45}Nb_{0,03}Sb_{0,01}Ca_{0,01}$$

$$Mo_{1,00}Pd_{0,00075}V_{0,55}Nb_{0,03}Sb_{0,01}Ca_{0,01}$$

$$Mo_{1,00}Pd_{0,00075}V_{0,45}Nb_{0,06}Sb_{0,01}\ Ca_{0,01}$$

$$Mo_{1,00}Pd_{0,0008}V_{0,55}Nb_{0,06}Sb0,01Ca_{0,01}$$

$$Mo_{1,00}Pd_{0,00085}V_{0,55}Nb_{0,06}Sb_{0,01}Ca_{0,01}$$

$$Mo_{1,00}Pd_{0,00075}V_{0,55}Nb_{0,09}Sb_{0,01}ca_{0,01}$$

$$Mo_{1,00}aPd_{0,0008}V_{0,50}Nb_{0,15}Te_{0,01}Ca_{0,01}$$

$$Mo_{1,00}Pd_{0,00075}V_{0,50}Nb_{0,09}W_{0,01}\ Pd_{0,0003}$$

Neben den Reaktionsgasen Ethan und Sauerstoff kann das Reaktionsgasgemisch Wasserdampf und Inertgase wie Stickstoff oder Kohlendioxid enthalten. Dabei kann in einem Wirbelschichtreaktor auch mit einer Reaktionsgaszusammensetzung oberhalb der Explosionsgrenze gearbeitet werden. Bevorzugt wird jedoch unter Inertgas-, Wasser-, und/oder Ethan-Überschuß gearbeitet. Wasserdampf beschleunigt die Oxidation von Ethan zu Essigsäure auf Kosten der Oxidation von Ethan zu Ethylen und hat somit eine Erhöhung der Selektivität zu Essigsäure zur Folge.

Das Verfahren wird bevorzugt bei niedrigen Temperaturen durchgeführt. Niedrige Temperaturen erhöhen die Selektivität der Oxidationsreaktion zu Gunsten der Essigsäure. Eine Temperaturbereich von 100 bis 500°C bei einem Reaktordruck von 1 bis 50 bar sind bevorzugt.

Die Dichte der Katalysatorpartikel kann zwischen 500 und 6000 kg/m³ liegen. Zur Durchführung des Verfahrens kann z. B. ein Wirbelschichtreaktor verwendet werden, wie er in US 5,300,684 beschrieben ist.

Das erfindungsgemäße Verfahren besitzt eine hohe Selektivität, dementsprechend können erhöhte Raum-Zeit-Ausbeuten bei der Oxidation von Ethan zur Essigsäure in einem Wirbelschichtreaktor, bei gemäßigten Temperaturen, erreicht werden. Weiterhin kann über die Verringerung der Blasengröße die störende Rückvermischung der Reaktionspartner in der Wirbelschicht vermindert werden. Dies führt ebenfalls zu einer erhöhten Selektivität der Reaktion, wodurch die Raumzeitausbeute an Essigsäure steigt.

In einer besonderen Ausführungsform, insbesondere wenn hohe Ethanumsätze gewünscht sind, kann die Reaktion im mehrstufigen Wirbelschichtreaktor mit eigener Gaszufuhr in jede Stufe, durchgeführt werden. In jeder Stufe beginnt daß Blasenwachstum erneut, so daß im Mittel kleinere Blasen als in einer einstufigen Wirbelschicht erreicht werden. Die kleinen Blasen können auch durch Einbauten, wie z. B. Gitter, in die Wirbelschicht erreicht werden. Zur Erzielung hoher Ethanumsätze bei gleichzeitig hoher Selektivität zu Essigsäure kann eine darüber hinausgehende verteilte Sauerstoffzufuhr in einzelne Stufen vorteilhaft sein.

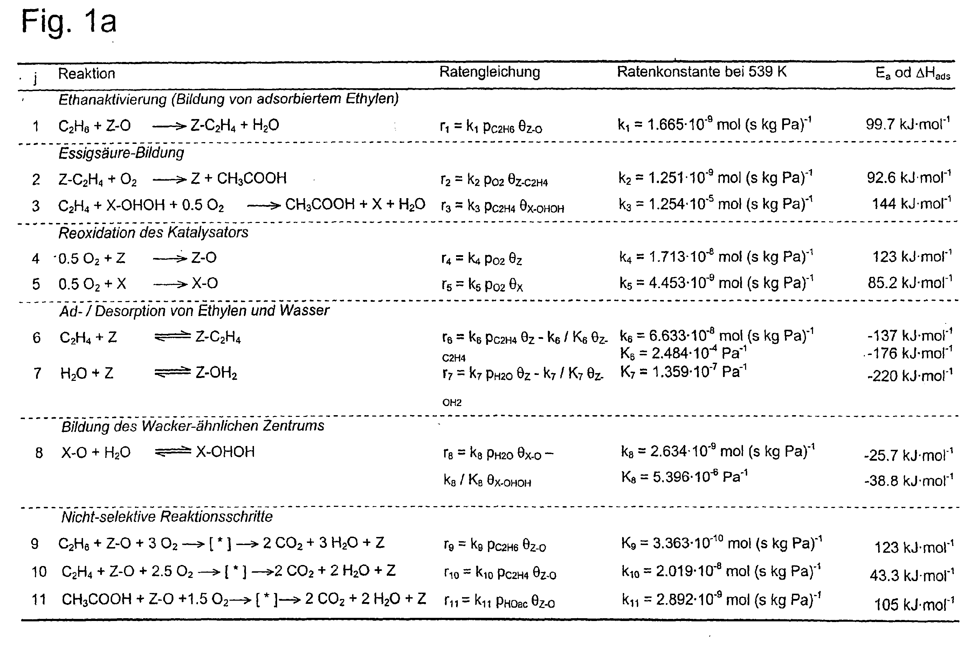

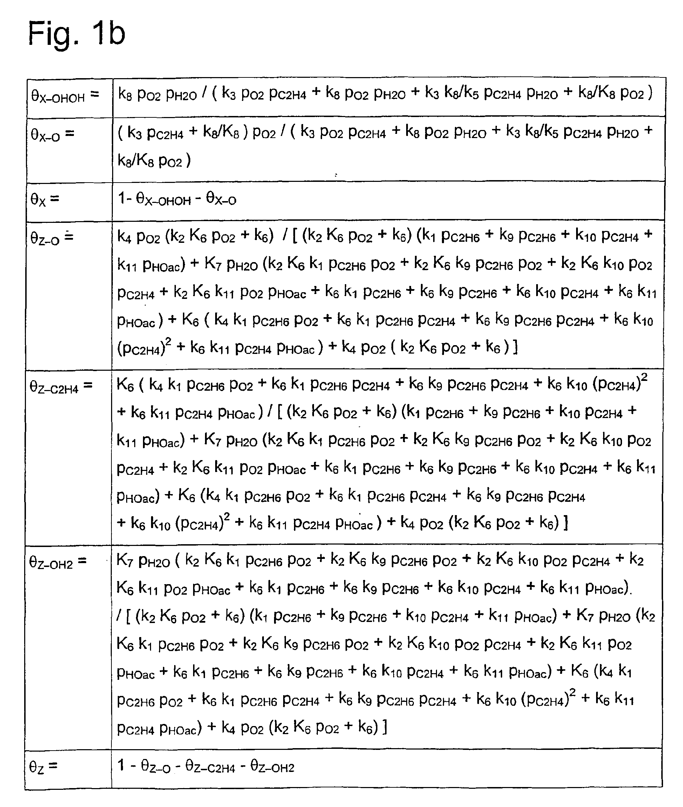

Die vorteilhaften Blasengrößen in einer fluiden Wirbelschicht und die entsprechenden Reaktionsparameter wurden aus Messergebnissen ermittelt, die bei Untersuchungen der katalytischen Gasphasenoxidation von Ethan im Festbett gewonnen wurden. Die daraus ermittelten kinetischen Parameter für die zugrundeliegende Reaktion sind in Fig. 1a und 1b zusammengefaßt. Die katalytische Gasphasenoxidation von Ethan wird von zwei unterschiedlichen katalytisch aktiven Stellen Z und X des Katalysators vermittelt. Z stellt eine Stelle dar, an der alle Oxidationsschritte ablaufen, nämlich die oxidative Dehydrierung von Ethan, die partielle und die vollständige Oxidation von Ethen und von Essigsäure. X ist eine Position am Katalysator, die nur in Anwesenheit von Wasser aktiv wird. Die aktivierte Form von X (X-OHOH) führt zur Umwandlung von Ethen zu Essigsäure über einen Mechanismus ähnlich der Wacker-Reaktion. Die Bildung von Essigsäure erfolgt somit über zwei verschiedene Reaktionswege - über die partielle Oxidation von Ethen an der Postion Z (Schritt 2 in Fig. 1 a) und über einen Mechanismus ähnlich der Wacker-Reaktion an Position X (Schritt 3 in Fig. 1 a). Die benötigten kinetischen Werte zu den Intermediaten an der katalytischen Oberfläche während des steady-state-Zustandes Z, Z-OH$_2$, Z-O, Z-CH$_2$H$_4$, X-OHOH und X-O wurden analytisch ermittelt und sind in Tabelle X angegeben.

Die so ermittelten Werte wurden zur Simulation des Wirbelschichtreaktors nach dem Bubble-Assemblage-Modell (BAM) benutzt (K. Kato, C.H. Wen, Chem. Eng. Sci. 24,1351-1368 (1969)).

Das Modell beschreibt das Wirbelschichtbett als ein zweiphasiges System, das aus einer katalysatorarmen Blasenphase und einer katalysatorreichen Emulsionsphase besteht; der zweiphasige Aufbau eines solchen Reaktionsgemisches aus Partikeln in einer fluiden Wirbelschicht bei Gaszufuhr kann mit bloßem Auge verifiziert werden. Da die Emulsionsphase einen großen Anteil an Feststoff enthält, findet dort die katalytische Reaktion statt, während in der Blasenphase aufgrund der geringen Konzentration des katalytischen Materials fast keine Reaktion stattfindet. Das Modell des Wirbelschichtreaktors muß deshalb den Übergang von Reaktanten und Produkten zwischen der Blasen- und der Emulsionsphase beschreiben und zugleich das Wachsen der Blasen mit steigendem Abstand vom Gasverteiler berücksichtigen, ein Effekt der aus fluiden Wirbelschichten gut bekannt ist. Aus diesem Grund wurde das Wirbelschichtbett in Segmente mit der Höhe aufgeteilt, die dem örtlichen Blasendurchmesser entspricht. Der Blasendurchmesser, die Geschwindigkeit der Blasen, die Ausdehnung des Bettes und die örtlichen Volumenfraktionen der Phasen wurden nach Werther (J. Werther, Chem. Eng. Sci. 47 (9-11), 2457-2462 (1992)) und Murray (J.D. Murray, J. Fluid. Mech. 21, 465 (1965)) berechnet. Für beide, Blasen- und Emulsionsphase, wurde jedes Segment als ein idealer Rührkessel beschrieben. Isotherme Reaktionsbedingungen können aufgrund der guten Wärmeabfuhr aus Wirbelschichtreaktoren annähernd eingestellt werden; daher wurde der Reaktor in der Modellierung als isotherm betrachtet.

Die Massengleichung für beide Phasen wird durch folgende Gleichungen gegeben:

Emulsionsphase:

$$0 = A_T u_{mf} \left[ c_{E,i,k-1} - c_{E,i,k} \right] + k_{BE,k} V_{B,k} \left[ c_{B,i,k} - c_{E,i,k} \right] + \sum_j \nu_{i,j} r_{E,i,k} (1 - \varepsilon_{mf}) \rho_{cat} V_{E,k}$$

Eq. 1

Blasenphase:

$$0 = A_T u_{mf} \left( u_k - u_{mf} \right) \left[ c_{B,i,k-1} - c_{B,i,k} \right] - k_{BE,k} V_{B,k} \left[ c_{B,i,k} - c_{E,i,k} \right]$$

Eq. 2

Die Gleichungen können für jede Reaktionskomponente i und jedes Segment k z.B. mit Hilfe der Wegstein-Methode gelöst werden. Der Stoff-Übergang zwischen der Blasen- und der Emufsionsphase, der durch den Koeffizient. $K_{BE,i,k}$ ausgedrückt wird, kann quantitativ nach einer Korrelation von Sit und Grace (S.P., Sit, J.R. Grace, Chem. Eng. Sci. 36, 327-335 (1981)) ermittelt werden. Die minimale Wirbelschichtgeschwindigkeit $u_{mf}$ wird wie in Wen et al. (C. Y. Wen, Y. H. Yu, AIChE J. 12, 610 (1966)) beschrieben abgeleitet.

Mit Hilfe des hier entwickelten Modells kann die direkte katalytische Oxidation von Ethan zu Essigsäure in einer fluiden Wirbelschicht weitgehend beschrieben werden.

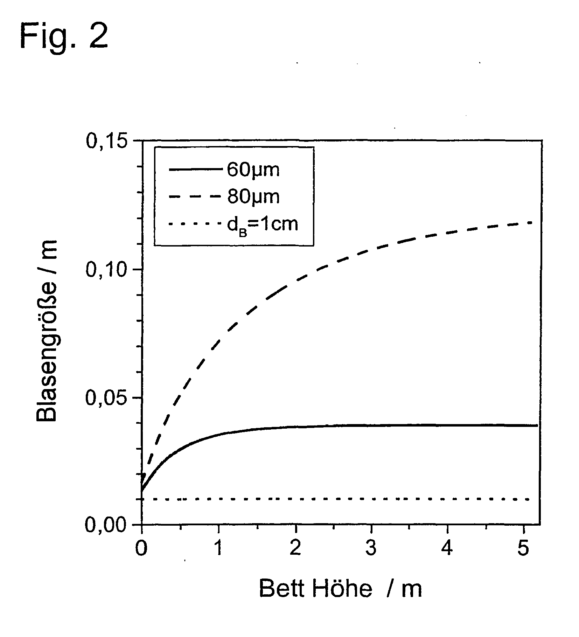

Die Ergebnisse werden in den Fig. 2 bis 7 dargelegt. $\tau_m$ steht in den Figuren und Tabellen für die modifizierte Raumzeit, die sich aus dem Quotienten aus Katalysatormasse und Volumenstrom bei den jeweiligen Reaktionsbedingungen ergibt.

Fig. 2 zeigt den Blasendurchmesser als Funktion der Höhe über der Gaszufuhr für unterschiedliche Partikeldurch-messer von 60 $\mu$m bzw. 80 $\mu$m

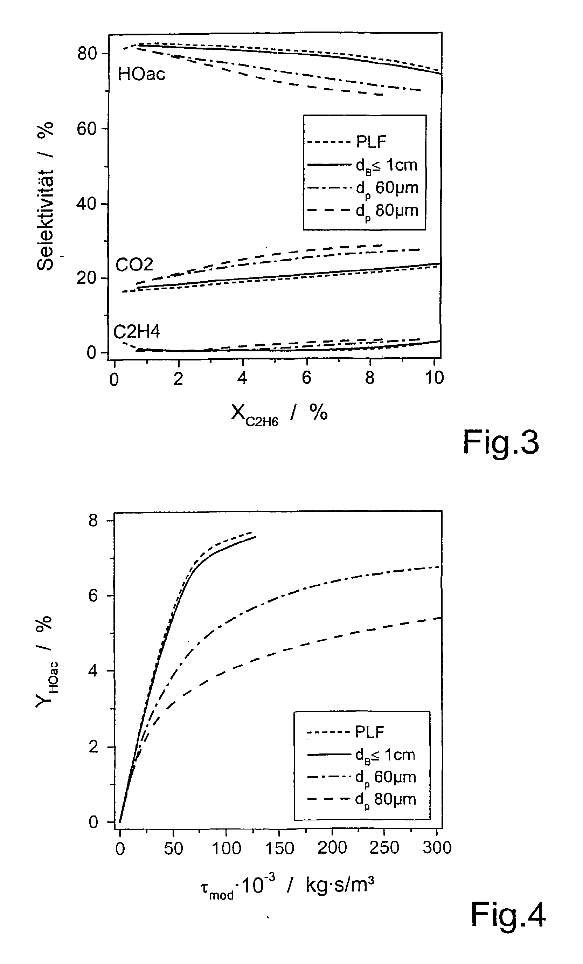

Fig. 3 zeigt die Umsatz-Selektivitätskurven und Fig. 4 die Ausbeute an Essigsäure in Abhängigkeit der Verweilzeit für verschiedene Partikel- bzw. Blasendurchmesser bei folgenden Reaktionsbedingungen: T = 260°C, $P_{tot}$ =16 bar, Edukt-Zusammensetzung $C_2H_6$ : $O_2$ : $H_2O$ : $N_2$ = 40 : 8 : 20 : 32.

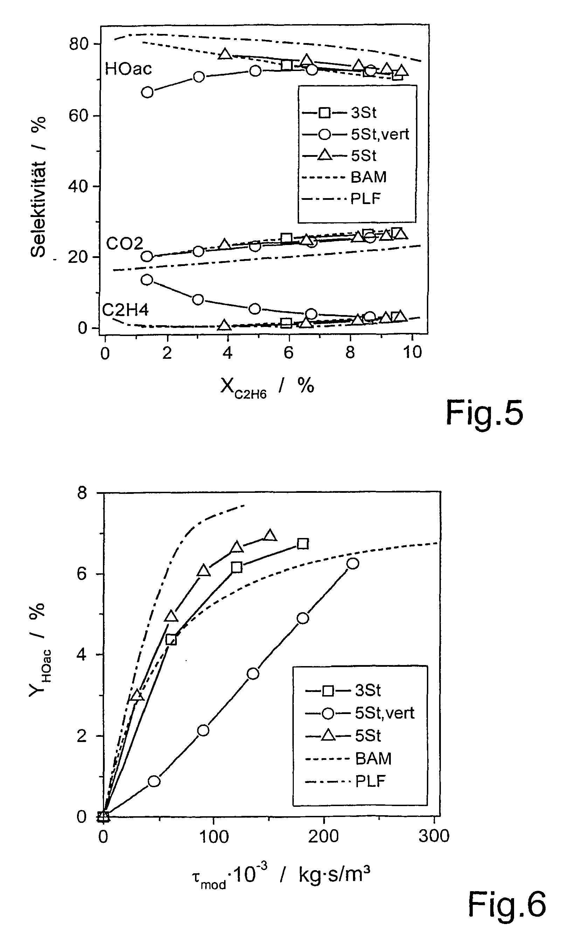

Fig. 5 zeigt die Umsatz-Selektivitätskurven und Fig. 6 die Ausbeute an Essigsäure in Abhängigkeit der Verweilzeit für mehrstufige Wirbelschichten (s.o.); die Punkte geben die Zusammensetzung am Ausgang jeder Stufe an; (Re-aktionsbedingungen: T = 260°C, $P_{tot}$ =16 bar, $d_p$ = 60 $\mu$m, Edukt-Zusammensetzung $C_2H_6$ : $O_2$ : $H_2O$ : $N_2$ = 40 : 8 : 20 : 32 (bei verteilter Sauerstoffzufuhr in der ersten Stufe: 40 : 1,6 : 20 : 32)).

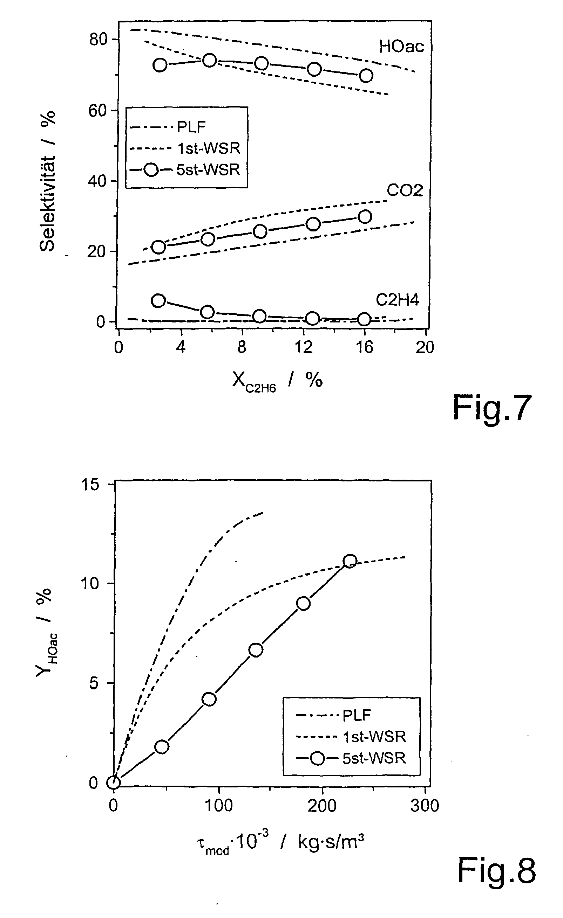

Fig. 7 zeigt die Umsatz-Selektivitätskurven und Fig. 8 die Ausbeute an Essigsäure in Abhängigkeit der Verweilzeit für Festbett-, 1-stufige Wirbelschicht- und 5-stufige Wirbelschicht mit verteilter Sauerstoffzufuhr; die Punkte geben die Zusammensetzung am Ausgang jeder Stufe an; (Reaktionsbedingungen: T = 260°C, $P_{tot}$ =16 bar, $d_p$ = 60 $\mu$m, Edukt-Zusammensetzung $C_2H_6$ : $O_2$: $H_2O$ : $N_2$ = 40 : 16 : 20 : 32 (bei verteilter Sauerstoffzufuhr in der 1. Stufe: 40 :

3,2 : 20 : 32)).

Beispiele

Die vorliegenden Berechnungen wurden durchgeführt unter Annahme eines Reaktordurchmessers $D_i$ von 4 m, einer Partikelgröße von 60 $\mu$m bzw. 80 $\mu$m (nach der Geldart Klassifikation A), einer Dichte der Partikel von 3100 kg/m3 und einer Porosität des Wirbelschicht-Reaktorbetts von 0,5 bei minimaler Fluidisierungsgeschwindigkeit. Die entsprechende minimale Fluidisierungsgeschwindigkeit betrug 3,3 m/s (bei 60$\mu$m) bzw. 5,85 m/s (bei 80$\mu$m). Um bei der Simulation vergleichbare fluid-dynamische Bedingungen im Reaktor zu erreichen, wurde die Wirbelschichtzahl $u/u_{mf}$ = 8,0 im Reaktor für alle Simulationsbedingungen konstant gehalten (unterschiedliche Temperatur, unterschiedliche Zusammensetzung des zugeführten Gasstroms), indem die Gas-Geschwindigkeit am Reaktoreinlaß zwischen 4,67 m$^3$/s und 5,40 m$^3$/s angepaßt wurde. Als Gasverteiler wurde ein Düsenplattenverteiler mit 800 Öffnungen pro m$^2$ angenommen.

Bei der Berechnung des mehrstufigen Wirbelschichtreaktors ohne verteilte Zufuhr von Sauerstoff wurde jede Stufe als ein einzelner Wirbelschichtreaktor mit eigenem Gasverteiler modelliert. Bei der mehrstufigen Wirbelschicht mit verteilter Sauerstoffzufuhr wird dem Gasstrom vor jeder Stufe der gleiche Sauerstoffstrom zugegeben. Dadurch nimmt der Volumenstromes von Stufe zu Stufe zu, so daß sich die Wirbelschichtzahl geringfügig von 7,5 auf 8,5 erhöht. Der Volumenstrom wurde so gewählt, daß die Wirbelschichtzahl in der mittleren Stufe der Wirbelschicht $u/u_{mf}$ = 8 beträgt.

In den Figuren sind als Vergleich die Simulationsergebnisse für einen idealen isothermen Festbettreaktor bei gleichen Bedingungen angegeben (Abkürzung: PLF). Die in den Figuren und im Anhang genannte modifizierte Verweilzeit $\tau_{mod}$ ist definiert als Masse Katalysator dividiert durch den Volumenstrom am Reaktoreingang bei den vorgegebenen Reaktionsbedingungen.

Beispiel 1: Einfluß der Partikelgröße

Für zwei verschiedene Partikeldurchmesser, 60 $\mu$m und 80 $\mu$m, wurden Simulationsrechnungen durchgeführt. Im Fall der 60 $\mu$m großen Partikel werden kleinere Blasen von maximal 4 cm Durchmesser gebildet, während bei den 80$\mu$m Partikeln Blasen bis zu 12 cm Durchmesser gebildet werden.

Die Auswirkung des besseren Stoffübergangs und der geringeren Rückvermischung bei kleinen Blasen auf die Selektivitäten und die Ausbeute an Essigsäure zeigen Fig. 3 und 4. Zusätzlich zeigen Fig. 3 und 4 das bei einem Blasendurchmesser von $d_B$ = 1 cm die erzielbaren Ausbeuten gleich denen im Festbettreaktor werden.

Die Ergebnisse zeigen weiterhin, daß bei gleichem Umsatz mit abnehmender Blasengröße die Selektivität zu Essigsäure ansteigt. Bei Verkleinerung des Partikeldurchmessers steigt außerdem die Ausbeute an Essigsäure $Y_{HOac}$ und auch die Raumzeitausbeute an (Fig. 4).

Das Beispiel zeigt, daß bei einer maximalen Blasengröße $d_B$ von 1 cm im Wirbelschichtreaktor und Festbettreaktor nahezu gleiche Ergebnisse bzgl. der Selektivität zu Essigsäure und der Raumzeitausbeute erhalten werden, da bei dieser Blasengröße praktisch keine Hemmung des Stoffübergangs zwischen Blasen und Emulsionsphase auftritt und zusätzlich die Rückvermischung reduziert wird.

Somit kann gezeigt werden, daß die geringe Selektivität und Raumzeitausbeute zumindest zum Teil auf die unerwünschte Rückvermischung in der Wirbelschicht und auf den langsamen Stofftransport zwischen der Blasen- und der Emulsionsphase zurückzuführen ist, der dazu führt, daß der Sauerstoff aus den Blasen nur langsam in die Emulsionsphase gelangt. Eine hohe Sauerstoffkonzentration in den Blasen führt zusätzlich zu verstärkter Kohlendioxid-Bildung durch unselektive Weiteroxidation der Essigsäure und damit zu einer Verringerung der Essigsäure-Selektivität.

Beispiel 2: Mehrstufiger Wirbelschichtreaktor

Eine zusätzliche Möglichkeit die unerwünschte Rückvermischung in der Wirbelschicht zu reduzieren ist - neben der Reduzierung der Partikelgröße - der Einsatz eines mehrstufigen Wirbelschichtreaktors. Durch die Gasverteiler zu Beginn jeder Stufe sind die Blasen gemittelt über den Wirbelschichtreaktor kleiner als in einer einstufigen Wirbelschicht, da in jeder Stufe das Blasenwachstum neu beginnt.

In Fig. 5 und 6 sind die Ergebnisse für verschiedene mehrstufige Wirbelschichtreaktoren dargestellt:

- "3St" - 3-stufiger Wirbelschichtreaktor mit 20 t Katalysator / Stufe, Höhe des fluidisierten Bettes pro Stufe 1,04 m

- "5St" - 5-stufiger Wirbelschichtreaktor mit 10 t Katalysator / Stufe, Höhe des fluidisierten Bettes pro Stufe 0,53 m

- "5St, vert" - 5-stufiger Wirbelschichtreaktor mit verteilter Sauerstoffzufuhr
  und
  15 t Katalysator / Stufe, Höhe des fluidisierten Bettes pro Stufe 0,78 m

Zum Vergleich wurden in Fig. 5 und 6 auch die Ergebnisse für den einstufigen Wirbelschichtreaktor (BAM) und den idealen Festbettreaktor (PLF) dargestellt.

Bei geringen Ethan-Umsätzen wird in der 5-stufigen Wirbelschicht mit verteilter Sauerstoffzufuhr Ethylen mit deutlich höherer Selektivität gebildet als in den anderen Wirbelschichtreaktoren. Dies ist auf die geringe Sauerstoffkonzentration zurückzuführen. Bei größeren Ethan-Umsatz dagegen sind die Unterschiede in den Selektivitäten zwischen den verschiedenen Wirbelschichtreaktoren gering (<2%). Die nach dem Festbettreaktor höchste Essigsäure-Selektivität von $S_{HOac}$ = 72,2 % wird in der 5-stufigen Wirbelschicht mit verteilter Sauerstoffzufuhr erhalten (bei $X_{C2H6}$= 8,6 %). Bei gleichem Ethan-Umsatz beträgt die Selektivität im Festbettreaktor $S_{HOac}$ = 77,5 %.

Die Ergebnisse in Figur 5 zeigen, daß durch Einsatz einer mehrstufigen Wirbelschicht die Raumzeitausbeute gegenüber einer einstufigen Wirbelschicht deutlich erhöht werden kann. Dagegen führt die verteilte Sauerstoffzufuhr zur Reduzierung der Raumzeitausbeute, da durch den niedrigeren Sauerstoffpartialdruck die Reaktionsgeschwindigkeit gegenüber den anderen Reaktoren herabgesetzt ist.

Die Simulation des 5-stufigen Wirbelschichtreaktors mit verteilter SauerstoffZufuhr wurde für eine höhere Sauerstoffkonzentration wiederholt, die einen höheren Ethan-Umsatz und damit eine höhere Essigsäure-Ausbeute erlaubt. Aufgrund des höheren Sauerstoff-Partialdruckes ist die Reaktionsgeschwindigkeit erhöht. Wie zuvor beträgt die Katalysatormasse 15 t/Stufe. Die Ergebnisse der Simulation zeigt Fig. 7 und 8.

Im Gegensatz zu den Ergebnissen mit der niedrigen Sauerstoffkonzentration (Fig. 5 und 6) zeigen die Umsatz-Selektivitätskurven in Fig. 7 und 8 deutlichere Unterschiede zwischen dem 1-stufigen und dem 5-stufigen Wirbelschichtreaktor. Durch die verteilte Sauerstoffzufuhr wird bei gleichem Ethan-Umsatz gegenüber dem einstufigen Wirbelschichtreaktor eine deutlich höhere Selektivität zu Essigsäure erhalten (bei $X_{C2H6}$ = 16 %: $S_{HOac,1-st}$ = 65,4 % bzw. $S_{HOac,5-st}$ = 69,7 %). Der Selektivitätsverlust in der Wirbelschicht gegenüber dem Festbett ist relativ gering ($S_{HOac,PLF}$ = 73,6 %). Wie zuvor ist die Raumzeitausbeute bei verteilter Sauerstoffzufuhr gegenüber dem Festbettreaktor reduziert; sie liegt jedoch auf dem Niveau des einstufigen Wirbelschichtreaktors.

In den folgenden Tabellen sind zu allen Figuren die Daten aufgeführt.

Tabelle 1: Umsatz- und Selektivitätsdaten zum Einfluß der Blasengröße bzw. des Partikeldurchmessers (Fig. 3 und Fig. 4)

| $\tau_m$ / kg·s·m$^{-3}$ | $X_{C2H6}$ /% | $X_{O2}$ /% | $S_{C2H4}$/% | $S_{HOac}$/% | $S_{CO2}$ /% |
|---|---|---|---|---|---|
| 60 μm Partikeldurchmesser | | | | | |
| 7540 | 1,19 | 11,19 | 0,28 | 80,33 | 19,39 |
| 15080 | 2,22 | 21,29 | 0,22 | 78,80 | 20,99 |
| 30159 | 3,88 | 37,99 | 0,30 | 76,67 | 23,03 |
| 60318 | 5,92 | 58,88 | 1,17 | 73,79 | 25,04 |
| 90477 | 7,13 | 71,35 | 1,77 | 72,28 | 25,94 |
| 150795 | 8,51 | 85,52 | 2,47 | 70,78 | 26,75 |
| 226193 | 9,31 | 93,75 | 2,91 | 69,96 | 27,13 |
| 301591 | 9,66 | 97,30 | 3,11 | 69,61 | 27,27 |
| 80 μm Partikeldurchmesser | | | | | |
| 4250 | 0,69 | 6,44 | 0,38 | 81,25 | 18,37 |
| 8499 | 1,31 | 12,43 | 0,27 | 80,00 | 19,74 |
| 16998 | 2,36 | 22,78 | 0,25 | 77,97 | 21,78 |
| 33996 | 3,65 | 35,94 | 1,01 | 74,89 | 24,10 |
| 50995 | 4,37 | 43,48 | 1,50 | 73,28 | 25,22 |
| 84991 | 5,29 | 53,10 | 1,98 | 71,69 | 26,33 |
| 127487 | 6,06 | 61,14 | 2,30 | 70,66 | 27,04 |
| 169982 | 6,64 | 67,00 | 2,52 | 70,02 | 27,46 |
| 254973 | 7,50 | 76,11 | 2,81 | 69,24 | 27,95 |
| Blasendurchmesser limitiert, $d_B$ = 1 cm | | | | | |
| 4250 | 0,72 | 6,59 | 0,69 | 81,99 | 17,32 |
| 8499 | 1,41 | 13,06 | 0,46 | 81,75 | 17,79 |

Tabelle fortgesetzt

| Blasendurchmesser limitiert, $d_B$ = 1 cm | | | | | |
|---|---|---|---|---|---|
| 16998 | 2,73 | 25,55 | 0,27 | 81,12 | 18,60 |
| 33996 | 5,11 | 48,46 | 0,19 | 79,80 | 20,01 |
| 50995 | 7,07 | 67,91 | 0,26 | 78,55 | 21,19 |
| 84991 | 9,40 | 91,07 | 1,72 | 75,55 | 22,74 |
| 127487 | 10,16 | 98,61 | 2,57 | 74,10 | 23,33 |
| als Vergleich: Plug-Flow-Reaktor | | | | | |
| 1700 | 0,28 | 2,48 | 2,52 | 81,21 | 16,27 |
| 4250 | 0,72 | 6,54 | 0,98 | 82,48 | 16,54 |
| 8499 | 1,42 | 12,98 | 0,51 | 82,53 | 16,96 |
| 16998 | 2,78 | 25,74 | 0,27 | 81,93 | 17,79 |
| 33996 | 5,23 | 49,22 | 0,18 | 80,52 | 19,30 |
| 50995 | 7,24 | 69,06 | 0,26 | 79,19 | 20,55 |
| 67993 | 8,75 | 84,07 | 0,93 | 77,52 | 21,55 |
| 84991 | 9,63 | 92,70 | 1,82 | 75,97 | 22,22 |
| 127487 | 10,28 | 99,18 | 2,58 | 74,65 | 22,76 |

Tabelle 2: Mehrstufiger Wirbelschichtreaktoren ohne Seitenströme (aller Sauerstoff wird in die erste Stufe gefahren) (Fig. 5 und Fig. 6)

| | $\tau_m$ / kg·s·m⁻³ | $X_{C2H6}$ /% | $X_{O2}$ /% | $S_{C2H4}$ /% | $S_{HOac}$ /% | $S_{CO2}$ /% |
|---|---|---|---|---|---|---|
| 3-stufiger Wirbelschichtreaktor | | | | | | |
| Stufe 1 | 60318 | 5,91 | 58,88 | 1,17 | 73,79 | 25,04 |
| Stufe 2 | 120636 | 8,55 | 85,61 | 2,09 | 71,86 | 26,06 |
| Stufe 3 | 180954 | 9,49 | 95,22 | 2,71 | 70,82 | 26,48 |
| 5-stufiger Wirbelschichtreaktor | | | | | | |
| Stufe 1 | 30159 | 3,88 | 37,98 | 0,30 | 76,67 | 23,03 |
| Stufe 2 | 60318 | 6,56 | 65,11 | 0,88 | 74,90 | 24,22 |
| Stufe 3 | 90477 | 8,23 | 81,65 | 1,59 | 73,42 | 24,99 |
| Stufe 4 | 120636 | 9,14 | 90,64 | 2,20 | 72,36 | 25,44 |
| Stufe 5 | 150795 | 9,62 | 95,30 | 2,57 | 71,73 | 25,69 |

Tabelle 3: Mehrstufiger Wirbelschichtreaktor mit verteilter Sauerstoffzufuhr (der Sauerstoffumsatz $X_{O2}$ ist pro Stufe berechnet) (Fig. 5 und Fig. 6)

| | $\tau_m$ / kg·s·m⁻³ | $X_{C2H6}$ /% | $X_{O2}$ /% | $S_{C2H4}$ /% | $S_{HOac}$ /% | $S_{CO2}$ /% |
|---|---|---|---|---|---|---|
| geringe Sauerstoffkonzentration | | | | | | |
| Stufe 1 | 45239 | 1,33 | 59,38 | 13,49 | 66,45 | 20,06 |
| Stufe 2 | 90477 | 3,02 | 57,95 | 7,90 | 70,64 | 21,45 |
| Stufe 3 | 135716 | 4,88 | 57,39 | 5,13 | 72,16 | 22,71 |
| Stufe 4 | 180954 | 6,73 | 57,06 | 3,62 | 72,48 | 23,90 |
| Stufe 5 | 226193 | 8,62 | 56,81 | 2,73 | 72,22 | 25,05 |
| erhöhte Sauerstoffkonzentration | | | | | | |
| Stufe 1 | 45239 | 2,47 | 58,47 | 5,99 | 72,77 | 21,24 |
| Stufe 2 | 90477 | 5,67 | 56,38 | 2,66 | 73,98 | 23,36 |

Tabelle fortgesetzt

| erhöhte Sauerstoffkonzentration | | | | | |
|---|---|---|---|---|---|
| Stufe 3 | 135716 | 9,10 | 55,10 | 1,42 | 73,09 | 25,49 |
| Stufe 4 | 180954 | 12,56 | 54,20 | 0,87 | 71,51 | 27,62 |
| Stufe 5 | 226193 | 15,96 | 53,49 | 0,60 | 69,69 | 29,72 |

Tabelle 4: Umsatz- und Selektivitätsdaten für den erhöhten Sauerstoffanteil; als Vergleich zum 5-stufigen Wirbelschichtreaktor mit verteilter Sauerstoffzufuhr sind hier Festbettreaktor und einstufiger Wirbelschichtreaktor aufgeführt. (Fig. 7 und Fig. 8)

| $\tau_m$ / kg·s·m$^{-3}$ | $X_{C2H6}$/% | $X_{O2}$/% | $S_{C2H4}$/% | $S_{HOac}$/% | $S_{CO2}$/% |
|---|---|---|---|---|---|
| einstufiger Wirbelschichtreaktor | | | | | |
| 7058 | 1,52 | 7,25 | 0,16 | 79,33 | 20,51 |
| 14115 | 2,90 | 14,18 | 0,11 | 77,15 | 22,74 |
| 28231 | 5,33 | 26,91 | 0,08 | 74,00 | 25,92 |
| 42346 | 7,37 | 38,02 | 0,07 | 71,82 | 28,11 |
| 56461 | 9,09 | 47,56 | 0,07 | 70,29 | 29,64 |
| 84692 | 11,71 | 62,35 | 0,16 | 68,35 | 31,49 |
| 141154 | 14,85 | 80,11 | 0,63 | 66,17 | 33,19 |
| 211730 | 16,77 | 90,97 | 1,09 | 64,89 | 34,02 |
| 282307 | 17,64 | 95,91 | 1,35 | 64,29 | 34,36 |
| Plug-Flow-Reaktor | | | | | |
| 2823 | 0,61 | 2,77 | 0,95 | 82,56 | 16,48 |
| 7058 | 1,58 | 7,27 | 0,37 | 82,56 | 17,07 |
| 14115 | 3,10 | 14,43 | 0,19 | 81,81 | 18,00 |
| 28231 | 6,04 | 28,64 | 0,10 | 80,10 | 19,80 |
| 56461 | 11,18 | 54,78 | 0,06 | 76,94 | 23,01 |
| 84692 | 15,22 | 76,56 | 0,06 | 74,36 | 25,58 |
| 112923 | 18,00 | 92,00 | 0,32 | 72,28 | 27,39 |
| 141154 | 19,11 | 98,18 | 0,95 | 70,86 | 28,19 |

**Patentansprüche**

1. Verfahren zur Herstellung von Essigsäure durch Oxidation von Ethan mit motekutarem Sauerstoff in einer Wirbelschicht aus Katalysatorpartikeln **dadurch gekennzeichnet, daß** der Partikeldurchmesser von mindestens 70% der eingesetzten Katalysatorpartikel kleiner oder gleich 80 μm ist, die Dichte der eingesetzten Katalysatorpartikel im Wirbelschichtreaktor zwischen 500 kg/m$^3$ und 6000 kg/m$^3$ liegt und die Wirbelschichtzahl größer oder gleich 1 ist, wobei der Gasvolumenstrom so gewählt wird, dass der Blasendurchmesser des eingespeisten Reaktionsgasgemisches in dem Wirbelschichtreaktor kleiner als 12 cm ist.

2. Verfahren gemäß Anspruch 1 **dadurch gekennzeichnet, daß** der Gasvolumenstrom so eingestellt wird, dass der Blasendurchmesser des eingespeisten Reaktionsgasgemisches kleiner als 5 cm ist.

3. Verfahren gemäß Anspruch 1 oder 2 **dadurch gekennzeichnet, daß** der Partikeldurchmesser von 10 % bis 60 % der Katalysatorpartikel unter 60 μm liegt.

4. Verfahren gemäß einem der Ansprüche 1 bis 3 **dadurch gekennzeichnet, daß** 20 bis 50 % der Partikel einen Durchmesser von kleiner 40 μm besitzen.

5. Verfahren gemäß einem der Ansprüche 1 bis 4 **dadurch gekennzeichnet, daß** die Katalysatorpartikel innerhalb eines Partikeldurchmesserbereichs von 10 μm bis 40 μm liegen.

6. Verfahren gemäß einem der vorhergehenden Ansprüche **dadurch gekennzeichnet, daß** der Wirbelschichtzahl zwischen 1 und 10 liegt.

7. Verfahren nach einem der vorhergehenden Ansprüche, **dadurch gekennzeichnet, daß** ein Katalysator der allgemeinen Formel (I) verwendet wird.

$$Mo_aPd_bX_cY_d \qquad (I)$$

wobei

X für eines oder mehrere der Elemente ausgewählt aus der Gruppe Cr, Mn, Nb, Ta, B, Ti, V, Te, Re und W steht. Y für eines oder mehrere der Elemente ausgewählt aus der Gruppe B, Al, Ga, In, Pt, Zn, Cd, Bl, Ce, Co, Rh, Ir, Cu, Ag, Au, Fe, Ru, Cs, Mg, Ca, Sr, Ba, Li, K, Na, Rb, Be, Nb, Zr, Hf, Ni, P, Pb, Sb, Si, Sn , Tl, und U steht und die Indices a, b, c und d für die Grammatomverhältnisse der entsprechenden Elemente stehen, wobei bezogen auf a=1, b zwischen 0,0001 und 0,01 c zwischen 0.1 und 1 und d zwischen 0 und 1 liegen, wobei X und/oder Y für mehrere Elemente stehen können.

8. Verfahren gemäß einem der vorhergehenden Ansprüche, **dadurch gekennzeichnet, daß** der Katalysator

$$Mo_{1,0}Pd_{0,0005}V_{0,25}Nb_{0,12}$$

$$Mo_{1,00}Pd_{0,0005}V_{0,45}Nb_{0,03}Sb_{0,01}Ca_{0,01}$$

$$Mo_{1,00}Pd_{0,0005}V_{0,45}Nb_{0,03}Sb_{0,01}Ca_{0,01}K_{0,015}$$

$$Mo_{1,00}Pd_{0,00075}V_{0,45}Nb_{0,03}Sb_{0,01}Ca_{0,01}$$

$$Mo_{1,00}Pd_{0,00075}V_{0,55}Nb_{0,03}Sb_{0,01}Ca_{0,01}$$

$$Mo_{1,00}Pd_{0,00075}V_{0,45}Nb_{0,06}Sb_{0,01}Ca_{0,01}$$

$$Mo_{1,00}Pd_{0,0008}V_{0,55}Nb_{0,05}Sb_{0,01}Ca_{0,01}$$

$$Mo_{1,00}Pd_{0,00085}V_{0,55}Nb_{0,00}Sb_{0,01}\ Ca_{0,01}$$

$$Mo_{1,00}Pd_{0,00075}V_{0,55}Nb_{0,09}Sb_{0,01}Ca_{0,01}$$

$$Mo_{1,00}Pd_{0,0008}V_{0,50}Nb_{0,15}Te_{0,01}Ca_{0,01}$$

$$Mo_{1,00}Pd_{0,00075}V_{0,50}Nb_{0,09}W_{0,01}Pd_{0,0003}$$

ist.

9. Verfahren nach einem der vorhergehenden Ansprüche, **dadurch gekennzeichnet, daß** die Reaktion bei einer Temperatur im Bereich zwischen 100 und 500°C ausgeführt wird.

10. Verfahren gemäß einem der vorhergehenden Anspruche, **dadurch gekennzeichnet, daß** die Reaktion bei einem Reaktordruck im Bereich von 1 bis 50 bar ausgeführt wird.

11. Verfahren nach einem der vorhergehenden Ansprüche, **dadurch gekennzeichnet, daß** dem Reaktionsgasgemisch neben Ethan und molekularem Sauerstoff ein Inertgas, Kohlendioxid und/oder Wasserdampf beigemischt wird.

12. Verfahren gemäß einem der vorhergehenden Ansprüche, **dadurch gekennzeichnet, daß** der Wirbelschichtreaktor aus mehreren Reaktorstufen bzw. Reaktotzonen besteht.

13. Verfahren gemäß einem der vorhergehenden Ansprüche, **dadurch gekennzeichnet, daß** ein, oder mehrere Reaktanten oder Wasser in verschiedene Zonen bzw. Stufen eines ein- oder mehrstufigen Reaktors zugeführt werden.

**Claims**

1. A process for preparing acetic acid by oxidation of ethane by means of molecular oxygen in a fluidized bed of catalyst particles, wherein the particle diameter of at least 70% of the catalyst particles used is less than or equal to 80 $\mu$m, the density of the catalyst particles used in the fluidized-bed reactor is in the range from 500 kg/m$^3$ to 6 000 kg/m$^3$, the fluidized bed index is greater than or equal to 1 and the gas volume flow is selected so that the bubble diameter of the reaction gas mixture fed in in the fluidized-bed reactor is less than 12 cm.

2. A process as claimed in claim 1, wherein the gas volume flow is set so that the bubble diameter of the reaction gas mixture fed in is less than 5 cm.

3. A process as claimed in claim 1 or 2, wherein the particle diameter of from 10% to 60% of the catalyst particles is less than 60 $\mu$m.

4. A process as claimed in any of claims 1 to 3, wherein from 20 to 50% of the particles have a diameter of less than 40 $\mu$m.

5. A process as claimed in any of claims 1 to 4, wherein the catalyst particles are within a particle diameter range from 10 $\mu$m to 40 $\mu$m.

6. A process as claimed in any of the preceding claims, wherein the fluidized bed index is in the range of from 1 to 10.

7. A process as claimed in any of the preceding claims, wherein the catalyst used has the formula (I),

$$Mo_aPd_bX_cY_d \qquad (I)$$

where
X is one or more elements selected from the group consisting of Cr, Mn, Nb, Ta, B, Ti, V, Te, Re and W,
Y is one or more elements selected from the group consisting of B, Al, Ga, In, Pt, Zn, Cd, Bi, Ce, Co, Rh, Ir, Cu, Ag, Au, Fe, Ru, Cs, Mg, Ca, Sr, Ba, Li, K, Na, Rb, Be, Nb, Zr, Hf, Ni, P, Pb, Sb, Si, Sn , Tl and U and
the indices a, b, c and d are the gram atom ratios of the corresponding elements, where, based on a = 1, b is in the range from 0.0001 to 0.01, c is in the range from 0.1 to 1 and d is in the range from 0 to 1, where X and/or Y may represent a plurality of elements.

8. A process as claimed in any of the preceding claims, wherein the catalyst is

$$Mo_{1.0}Pd_{0.0005}V_{0.25}Nb_{0.12}$$

$$Mo_{1.00}Pd_{0.0005}V_{0.45}Nb_{0.03}Sb_{0.01}Ca_{0.01}$$

$$Mo_{1.00}Pd_{0.0005}V_{0.45}Nb_{0.03}Sb_{0.01}Ca_{0.01}K_{0.015}$$

$$Mo_{0.100}Pd_{0.00075}V_{0.45}Nb_{0.03}Sb_{0.01}Ca_{0.01}$$

$$Mo_{1.00}Pd_{0.00075}V_{0.55}Nb_{0.03}Sb_{0.01}Ca_{0.01}$$

$$Mo_{1.00}Pd_{0.00075}V_{0.45}Nb_{0.06}Sb_{0.01}Ca_{0.01}$$

$$Mo_{1.00}Pd_{0.0008}V_{0.55}Nb_{0.06}Sb_{0.01}Ca_{0.01}$$

$$Mo_{1.00}Pd_{0.00085}V_{0.55}Nb_{0.06}Sb_{0.01}Ca_{0.01}$$

$$Mo_{1.00}Pd_{0.0075}V_{0.55}Nb_{0.09}Sb_{0.01}Ca_{0.01}$$

$$Mo_{1.00}Pd_{0.0008}V_{0.50}Nb_{0.15}Te_{0.01}Ca_{0.01}$$

$$Mo_{1.00}Pd_{0.00075}V_{0.50}Nb_{0.09}W_{0.01}Pd_{0.0003}.$$

9. A process as claimed in any of the preceding claims, wherein the reaction is carried out at a temperature in the range from 100 to 500°C.

10. A process as claimed in any of the preceding claims, wherein the reaction is carried out at a reactor pressure in the range from 1 to 50 bar.

11. A process as claimed in any of the preceding claims, wherein not only ethane and molecular oxygen but also an inert gas, carbon dioxide and/or water vapor are present in the reaction gas mixture.

12. A process as claimed in any of the preceding claims, wherein the fluidized-bed reactor has a plurality of reaction stages or reaction zones.

13. A process as claimed in any of the preceding claims, wherein one or more reactants or water are fed into various zones or stages of a single-stage or multistage reactor.

**Revendications**

1. Procédé pour la préparation d'acide acétique par oxydation de l'éthane par l'oxygène moléculaire dans un lit fluidisé de particules de catalyseur **caractérisé en ce que** le diamètre de particule d'au moins 70 % des particules du catalyseur utilisé est inférieur ou égal à 80 $\mu$m, la densité des particules du catalyseur utilisées dans le réacteur à lit fluidisé se situe entre 500 kg/m$^3$et 6000 kg/m$^3$ et le nombre de lits fluidisés est supérieur ou égal à 1, le volume du flux gazeux étant choisi de façon telle que le diamètre des bulles du mélange réactionnel apporté au réacteur à lit fluidisé soit inférieur à 12 cm.

2. Procédé selon la revendication 1, **caractérisé en ce que** le volume du flux gazeux est réglé de façon telle que le diamètre des bulles du mélange réactionnel apporté soit inférieur à 5 cm.

3. Procédé selon la revendication 1 ou 2, **caractérisé en ce que** le diamètre de particule de 10 % à 60 % des particules du catalyseur est inférieur à 60 $\mu$m.

4. Procédé selon l'une des revendication 1 à 3, **caractérisé en ce que** 20 à 50 % des particules présentent un diamètre inférieur à 40 $\mu$m.

5. Procédé selon l'une des revendications 1 à 4, **caractérisé en ce que** les particules du catalyseur se situent dans un domaine de diamètres de particules de 10 $\mu$m à 40 $\mu$m.

6. Procédé selon l'une des revendications précédentes **caractérisé en ce que** le nombre de lits fluidisés est compris entre 1 et 10.

7. Procédé selon l'une des revendications précédentes, **caractérisé en ce qu'**on emploie un catalyseur de formule (I)

$$MO_aPd_bX_cY_d \qquad (I)$$

dans laquelle

X représente un ou plusieurs des éléments pris dans le groupe comprenant Cr, Mn, Nb, Ta, B, Ti, V, Te, Re et W, Y représente un ou plusieurs éléments pris dans le groupe comprenant B, Al, Ga, In, Pt, Zn, Cd, Bi, Ce, Co, Rh, Ir, Cu, Ag, Au, Fe, Ru, Cs, Mg, Ca, Sr, Ba, Li, K, Na, Rb, Be, Nb, Zr, Hf, Ni, P, Pb, Sb, Si, Sn, Ti et U, et

les indices a, b, c et d correspondent aux rapports en gramme-atomes des éléments correspondants, par rapport à a = 1, b est compris entre 0,0001 et 0,01, c est compris entre 0,1 et 1 et d est compris entre 0 et 1, X et Y peuvent représenter un ou plusieurs éléments.

8. Procédé selon l'une des revendications précédentes, **caractérisé en ce** le catalyseur répond à

$$Mo_{1,0}Pd_{0,0005}V_{0,25}Nb_{0,12}$$

$$Mo_{1,00}Pd_{0,0005}V_{0,45}Nb_{0,03}Sb_{0,01}Ca_{0,01}$$

$$Mo_{1,00}Pd_{0,0005}V_{0,45}Nb_{0,03}Sb_{0,01}Ca_{0,01}K_{0,015}$$

$$Mo_{1,00}Pd_{0,00075}V_{0,45}Nb_{0,03}Sb_{0,01}Ca_{0,01}$$

$$Mo_{1,00}Pd_{0,00075}V_{0,55}Nb_{0,03}Sb_{0,01}Ca_{0,01}$$

$$Mo_{1,00}Pd_{0,00075}V_{0,45}Nb_{0,06}Sb_{0,01}Ca_{0,01}$$

$$Mo_{1,00}Pd_{0,0008}V_{0,55}Nb_{0,06}Sb_{0,01}Ca_{0,01}$$

$$Mo_{1,00}Pd_{0,00085}V_{0,55}Nb_{0,06}Sb_{0,01}Ca_{0,01}$$

$$Mo_{1,00}Pd_{0,00075}V_{0,55}Nb_{0,09}Sb_{0,01}Ca_{0,01}$$

$$Mo_{1,00}Pd_{0,0008}V_{0,50}Nb_{0,15}Te_{0,01}Ca_{0,01}$$

$$Mo_{1,00}Pd_{0,00075}V_{0,50}Nb_{0,09}W_{0,01}Pd_{0,0003}$$

9.  Procédé selon l'une des revendications précédentes, **caractérisé en ce que** la réaction est mise en oeuvre à une température dans le domaine compris entre 100 et 500°C.

10. Procédé selon l'une des revendications précédentes, **caractérisé en ce que** la réaction est mise en oeuvre à une pression réactionnelle dans le domaine de 1 à 50 bar.

11. Procédé selon l'une des revendications précédentes, **caractérisé en ce qu'**on ajoute au mélange réactionnel, au côté de l'éthane et de l'oxygène moléculaire, un gaz inerte, le dioxyde de carbone et/ou la vapeur d'eau.

12. Procédé selon l'une des revendications précédentes, **caractérisé en ce que** le réacteur à lit fluidisé est constitué par un ou plusieurs étages réactionnels ou zones réactionnelles.

13. Procédé selon l'une des revendications précédentes, **caractérisé en ce qu'**on apporte un ou plusieurs réactants ou l'eau dans les zones ou étages différents d'un réacteur monoétagé ou multiétagé.

# Fig. 1a

| j | Reaktion | Ratengleichung | Ratenkonstante bei 539 K | $E_a$ od $\Delta H_{ads}$ |
|---|---|---|---|---|
| | *Ethanaktivierung (Bildung von adsorbiertem Ethylen)* | | | |
| 1 | $C_2H_6 + Z\text{-}O \longrightarrow Z\text{-}C_2H_4 + H_2O$ | $r_1 = k_1\, p_{C2H6}\, \theta_{Z\text{-}O}$ | $k_1 = 1.665 \cdot 10^{-9}$ mol (s kg Pa)$^{-1}$ | 99.7 kJ·mol$^{-1}$ |
| | *Essigsäure-Bildung* | | | |
| 2 | $Z\text{-}C_2H_4 + O_2 \longrightarrow Z + CH_3COOH$ | $r_2 = k_2\, p_{O2}\, \theta_{Z\text{-}C2H4}$ | $k_2 = 1.251 \cdot 10^{-9}$ mol (s kg Pa)$^{-1}$ | 92.6 kJ·mol$^{-1}$ |
| 3 | $C_2H_4 + X\text{-}OHOH + 0.5\,O_2 \longrightarrow CH_3COOH + X + H_2O$ | $r_3 = k_3\, p_{C2H4}\, \theta_{X\text{-}OHOH}$ | $k_3 = 1.254 \cdot 10^{-5}$ mol (s kg Pa)$^{-1}$ | 144 kJ·mol$^{-1}$ |
| | *Reoxidation des Katalysators* | | | |
| 4 | $0.5\,O_2 + Z \longrightarrow Z\text{-}O$ | $r_4 = k_4\, p_{O2}\, \theta_Z$ | $k_4 = 1.713 \cdot 10^{-8}$ mol (s kg Pa)$^{-1}$ | 123 kJ·mol$^{-1}$ |
| 5 | $0.5\,O_2 + X \longrightarrow X\text{-}O$ | $r_5 = k_5\, p_{O2}\, \theta_X$ | $k_5 = 4.453 \cdot 10^{-9}$ mol (s kg Pa)$^{-1}$ | 85.2 kJ·mol$^{-1}$ |
| | *Ad- / Desorption von Ethylen und Wasser* | | | |
| 6 | $C_2H_4 + Z \rightleftharpoons Z\text{-}C_2H_4$ | $r_6 = k_6\, p_{C2H4}\, \theta_Z - k_6/K_6\, \theta_{Z\text{-}C2H4}$ | $k_6 = 6.633 \cdot 10^{-8}$ mol (s kg Pa)$^{-1}$ <br> $K_6 = 2.484 \cdot 10^{-4}$ Pa$^{-1}$ | -137 kJ·mol$^{-1}$ <br> -176 kJ·mol$^{-1}$ |
| 7 | $H_2O + Z \rightleftharpoons Z\text{-}OH_2$ | $r_7 = k_7\, p_{H2O}\, \theta_Z - k_7/K_7\, \theta_{Z\text{-}OH2}$ | $K_7 = 1.359 \cdot 10^{-7}$ Pa$^{-1}$ | -220 kJ·mol$^{-1}$ |
| | *Bildung des Wacker-ähnlichen Zentrums* | | | |
| 8 | $X\text{-}O + H_2O \rightleftharpoons X\text{-}OHOH$ | $r_8 = k_8\, p_{H2O}\, \theta_{X\text{-}O} -$ <br> $k_8/K_8\, \theta_{X\text{-}OHOH}$ | $k_8 = 2.634 \cdot 10^{-9}$ mol (s kg Pa)$^{-1}$ <br> $K_8 = 5.396 \cdot 10^{-6}$ Pa$^{-1}$ | -25.7 kJ·mol$^{-1}$ <br> -38.8 kJ·mol$^{-1}$ |
| | *Nicht-selektive Reaktionsschritte* | | | |
| 9 | $C_2H_6 + Z\text{-}O + 3\,O_2 \longrightarrow [\,*\,] \longrightarrow 2\,CO_2 + 3\,H_2O + Z$ | $r_9 = k_9\, p_{C2H6}\, \theta_{Z\text{-}O}$ | $K_9 = 3.363 \cdot 10^{-10}$ mol (s kg Pa)$^{-1}$ | 123 kJ·mol$^{-1}$ |
| 10 | $C_2H_4 + Z\text{-}O + 2.5\,O_2 \longrightarrow [\,*\,] \longrightarrow 2\,CO_2 + 2\,H_2O + Z$ | $r_{10} = k_{10}\, p_{C2H4}\, \theta_{Z\text{-}O}$ | $k_{10} = 2.019 \cdot 10^{-8}$ mol (s kg Pa)$^{-1}$ | 43.3 kJ·mol$^{-1}$ |
| 11 | $CH_3COOH + Z\text{-}O + 1.5\,O_2 \longrightarrow [\,*\,] \longrightarrow 2\,CO_2 + 2\,H_2O + Z$ | $r_{11} = k_{11}\, p_{HOac}\, \theta_{Z\text{-}O}$ | $k_{11} = 2.892 \cdot 10^{-9}$ mol (s kg Pa)$^{-1}$ | 105 kJ·mol$^{-1}$ |

# Fig. 1b

| | |
|---|---|
| $\theta_{X\text{-}OHOH} =$ | $k_8\, p_{O2}\, p_{H2O} \,/\, (\, k_3\, p_{O2}\, p_{C2H4} + k_8\, p_{O2}\, p_{H2O} + k_3\, k_8/k_5\, p_{C2H4}\, p_{H2O} + k_8/K_8\, p_{O2}\,)$ |
| $\theta_{X\text{-}O} =$ | $(\, k_3\, p_{C2H4} + k_8/K_8\,)\, p_{O2} \,/\, (\, k_3\, p_{O2}\, p_{C2H4} + k_8\, p_{O2}\, p_{H2O} + k_3\, k_8/k_5\, p_{C2H4}\, p_{H2O} +$ $k_8/K_8\, p_{O2}\,)$ |
| $\theta_X =$ | $1 - \theta_{X\text{-}OHOH} - \theta_{X\text{-}O}$ |
| $\theta_{Z\text{-}O} =$ | $k_4\, p_{O2}\, (k_2\, K_6\, p_{O2} + k_6) \,/\, [\, (k_2\, K_6\, p_{O2} + k_6)\, (k_1\, p_{C2H6} + k_9\, p_{C2H6} + k_{10}\, p_{C2H4} +$ $k_{11}\, p_{HOac}) + K_7\, p_{H2O}\, (k_2\, K_6\, k_1\, p_{C2H6}\, p_{O2} + k_2\, K_6\, k_9\, p_{C2H6}\, p_{O2} + k_2\, K_6\, k_{10}\, p_{O2}$ $p_{C2H4} + k_2\, K_6\, k_{11}\, p_{O2}\, p_{HOac} + k_6\, k_1\, p_{C2H6} + k_6\, k_9\, p_{C2H6} + k_6\, k_{10}\, p_{C2H4} + k_6\, k_{11}$ $p_{HOac}\,) + K_6\, (\, k_4\, k_1\, p_{C2H6}\, p_{O2} + k_6\, k_1\, p_{C2H6}\, p_{C2H4} + k_6\, k_9\, p_{C2H6}\, p_{C2H4} + k_6\, k_{10}$ $(p_{C2H4})^2 + k_6\, k_{11}\, p_{C2H4}\, p_{HOac}\,) + k_4\, p_{O2}\, (\, k_2\, K_6\, p_{O2} + k_6\,)\,]$ |
| $\theta_{Z\text{-}C2H4} =$ | $K_6\, (\, k_4\, k_1\, p_{C2H6}\, p_{O2} + k_6\, k_1\, p_{C2H6}\, p_{C2H4} + k_6\, k_9\, p_{C2H6}\, p_{C2H4} + k_6\, k_{10}\, (p_{C2H4})^2$ $+ k_6\, k_{11}\, p_{C2H4}\, p_{HOac}\,) \,/\, [\, (k_2\, K_6\, p_{O2} + k_6)\, (k_1\, p_{C2H6} + k_9\, p_{C2H6} + k_{10}\, p_{C2H4} +$ $k_{11}\, p_{HOac}) + K_7\, p_{H2O}\, (k_2\, K_6\, k_1\, p_{C2H6}\, p_{O2} + k_2\, K_6\, k_9\, p_{C2H6}\, p_{O2} + k_2\, K_6\, k_{10}\, p_{O2}$ $p_{C2H4} + k_2\, K_6\, k_{11}\, p_{O2}\, p_{HOac} + k_6\, k_1\, p_{C2H6} + k_6\, k_9\, p_{C2H6} + k_6\, k_{10}\, p_{C2H4} + k_6\, k_{11}$ $p_{HOac}) + K_6\, (k_4\, k_1\, p_{C2H6}\, p_{O2} + k_6\, k_1\, p_{C2H6}\, p_{C2H4} + k_6\, k_9\, p_{C2H6}\, p_{C2H4}$ $+ k_6\, k_{10}\, (p_{C2H4})^2 + k_6\, k_{11}\, p_{C2H4}\, p_{HOac}\,) + k_4\, p_{O2}\, (k_2\, K_6\, p_{O2} + k_6)\,]$ |
| $\theta_{Z\text{-}OH2} =$ | $K_7\, p_{H2O}\, (\, k_2\, K_6\, k_1\, p_{C2H6}\, p_{O2} + k_2\, K_6\, k_9\, p_{C2H6}\, p_{O2} + k_2\, K_6\, k_{10}\, p_{O2}\, p_{C2H4} + k_2$ $K_6\, k_{11}\, p_{O2}\, p_{HOac} + k_6\, k_1\, p_{C2H6} + k_6\, k_9\, p_{C2H6} + k_6\, k_{10}\, p_{C2H4} + k_6\, k_{11}\, p_{HOac}).$ $/\, [\, (k_2\, K_6\, p_{O2} + k_6)\, (k_1\, p_{C2H6} + k_9\, p_{C2H6} + k_{10}\, p_{C2H4} + k_{11}\, p_{HOac}) + K_7\, p_{H2O}\, (k_2$ $K_6\, k_1\, p_{C2H6}\, p_{O2} + k_2\, K_6\, k_9\, p_{C2H6}\, p_{O2} + k_2\, K_6\, k_{10}\, p_{O2}\, p_{C2H4} + k_2\, K_6\, k_{11}\, p_{O2}$ $p_{HOac} + k_6\, k_1\, p_{C2H6} + k_6\, k_9\, p_{C2H6} + k_6\, k_{10}\, p_{C2H4} + k_6\, k_{11}\, p_{HOac}) + K_6\, (k_4\, k_1$ $p_{C2H6}\, p_{O2} + k_6\, k_1\, p_{C2H6}\, p_{C2H4} + k_6\, k_9\, p_{C2H6}\, p_{C2H4} + k_6\, k_{10}\, (p_{C2H4})^2 + k_6\, k_{11}$ $p_{C2H4}\, p_{HOac}) + k_4\, p_{O2}\, (k_2\, K_6\, p_{O2} + k_6)\,]$ |
| $\theta_Z =$ | $1 - \theta_{Z\text{-}O} - \theta_{Z\text{-}C2H4} - \theta_{Z\text{-}OH2}$ |

# Fig. 2

Fig.3

Fig.4

Fig.5

Fig.6

Fig.7

Fig.8